# EUROPEAN PATENT SPECIFICATION

(11) **EP 2 916 803 B1**
(45) Date of publication and mention of the grant of the patent: **16.08.2017**
(21) Application number: 13789301.2
(22) Date of filing: 08.11.2013
(51) Int. Cl.: A61K 8/365, A61Q 5/04, A61K 8/31, A61K 8/34, A61K 8/37, A61K 8/92, A61Q 5/06

(54) **COMPOSITION COMPRISING A DICARBONYL COMPOUND AND PROCESS FOR STRAIGHTENING THE HAIR USING THIS COMPOSITION**
ZUSAMMENSETZUNG MIT EINER DICARBONYLVERBINDUNG UND HAARGLÄTTUNGSVERFAHREN UNTER VERWENDUNG DIESER ZUSAMMENSETZUNG
COMPOSITION COMPRENANT UN COMPOSÉ DICARBONYLE ET PROCÉDÉ POUR LE LISSAGE DE CHEVEUX UTILISANT CETTE COMPOSITION

(30) Priority: 09.11.2012 FR 1260676
(43) Date of publication of application: 16.09.2015
(73) Proprietor: L'OREAL, 75008 Paris (FR)
(72) Inventor: DAUBRESSE, Nicolas, F-78170 La Celle Saint Cloud (FR); MORVAN, Christelle, F-95610 Eragny (FR)
(74) Representative: Leray, Noelle
(86) International application number: PCT/EP2013/073449
(87) International publication number: WO 2014/072491

(56) References cited:
- WO-A1-2007/135299
- WO-A1-2009/035970
- WO-A1-2012/105985
- WO-A2-2011/104282
- WO-A2-2012/010351
- DATABASE GNPD [Online] MINTEL; February 2012 (2012-02), "Moroccan Relaxing Treatment with Argan Oil", XP002703715, Database accession no. 1692744
- DATABASE GNPD [Online] MINTEL; October 2012 (2012-10), "Luxury Keratin Treatment", XP002703716, Database accession no. 1892615
- DATABASE GNPD [Online] MINTEL; January 2012 (2012-01), "Reduce Kit'30", XP002703717, Database accession no. 1704182

## Description

The present invention relates to a cosmetic composition, especially a hair composition, based on one or more particular dicarbonyl compounds and/or hydrates thereof and/or salts thereof, and also to a process for straightening keratin fibres, especially the hair, using this composition.

In the hair field, consumers wish to have available compositions which make it possible to introduce a temporary change to their head of hair, while targeting good remanence of the effect produced. In general, it is desirable for the change to withstand shampooing operations for a minimum of 15 days, indeed even more, depending on the nature of the said change.

Treatments already exist for modifying the colour or the shape of the hair and also, to some extent, the texture of the hair. One of the known treatments for modifying the texture of the hair consists of the combination of heat and of a formaldehyde-comprising composition. This treatment is especially effective for imparting a better appearance to damaged hair and/or for treating long hair and curly hair.

The action of formaldehyde is associated with its ability to crosslink proteins by reaction with the nucleophilic sites thereof. The heat used may be that of an iron (flat tongs or crimping iron), the temperature of which may generally be up to 200°C or more. However, it is increasingly sought to avoid the use of such substances, which may prove to be aggressive to the hair and other keratin materials.

Patent application WO 2011/104 282 thus proposed a novel process for semi-permanently straightening the hair, which consists in applying an α-keto acid solution to the hair for 15 to 120 minutes, then drying and, finally, straightening the head of hair with an iron at a temperature of about 200°C. The α-keto acid employed is preferably glyoxylic acid.

Patent application WO2012/105985 discloses a method of enhancing the removal of the natural curl from hair comprising the steps of applying an alkaline composition and then an acidic composition having a pH of less than 1,5 which comprises glyoxylic acid and/or glyoxylic amide.

However, it has been noted that the use of glyoxylic acid can result in some significant limitations; in particular, at high concentration it may not be well tolerated, in particular when the scalp is sensitive and/or irritated. Its volatility, amplified by the use of heat (iron), can also be a problem. Furthermore, cosmetic formulations having an acidic pH may impair the hair and/or impair its colour.

It is already known practice to use glyoxylic acid esters in hair compositions, in particular in hair dye compositions, as described in document DE19859722, and in reducing compositions, as described in document DE19860239.

However, the efficacy of these compositions is not yet sufficient.

These compositions comprising glyoxylic acid are in point of fact formulated at a very acidic pH. When they are based on emulsions, they may have stability problems, especially at high temperature, for instance during transportation or storage.

The instability of the formulations is troublesome in several respects. Besides the inconvenience for the user who is confronted with the use of diversely fluid formulations, this entails an increased risk of exposure to the acidity of these formulations, particularly for the region of the eyes if the product has become too liquid. Moreover, the fatty substances present in the formulations may rise to the surface of the compositions, clog the bottleware and lead to risks of overpressure or of non-uniformity on application. Finally, the cosmetic qualities obtained with the fresh formulations may be lost if the formulations are unstable, the cosmetic agents no longer being in equilibrium or only being present in one of the phases.

The aim of the invention is to develop a straightening/relaxing composition which is stable over time and which makes it possible to straighten/relax and/or reduce the volume of the hair in an efficient and persistent manner while limiting damage to the hair, while at the same time retaining comfort at the time of application for the user of the composition, but also for the hairdresser who applies it.

Thus, one subject of the present invention is a cosmetic composition comprising
- one or more dicarbonyl compounds corresponding to formula (I) below and/or hydrates thereof and/or salts thereof: 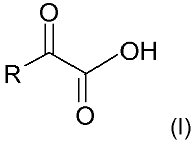 in which formula (I):
   R represents an atom or group chosen from i) hydrogen, ii) carboxyl -C(O) OH, iii) linear or branched C₁-C₆ alkyl which is optionally substituted, preferably with at least one hydroxyl -OH radical, carboxyl -C(O)-OH radical or halogen radical such as Br; iv) optionally substituted phenyl, v) optionally substituted benzyl, iv) and v) preferably being optionally substituted with at least one-OH or-C(O) OH radical; vi) an indolyl radical and vii) an imidazolylmethyl radical and tautomers thereof such as 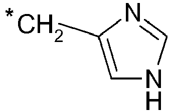 with * representing the part linked to the rest of the molecule, the dicarbonyl compounds corresponding to formula (I) and/or hydrates thereof and/or salts thereof being present in an amount ranging from 3 to 15% by weight relative to the total weight of the composition,
- one or more cationic surfactants,
- one or more fatty substances, preferably non-liquid fatty substances, and preferably solid fatty substances, the non-liquid fatty substances being present in an amount ranging from 1% to 30% by weight relative to the total weight of the composition,
   the composition having a pH of less than or equal to 4.

A subject of the invention is also a process for straightening keratin fibres, especially the hair, which comprises the application to the hair of the composition of the invention, followed by a straightening step using a straightening iron at a temperature of at least 150°C, preferably ranging from 150 to 250°C.

The composition of the invention is stable. The composition of the invention and the process for treating keratin fibres using it allow good straightening of keratin fibres while limiting damage to these keratin fibres, even when the application of the composition is followed by a heat treatment, in particular using a hair-straightening iron, and have an appreciated wear quality, in particular without excessive vaporization of the composition at the time of straightening. The composition and the process for treating keratin fibres according to the invention also make it possible to limit the change in the colour of the fibres and also the problems of breaking of the fibres such as the hair. The composition and the process of the invention will also improve the physical properties of the hair, by reducing the frizziness effect in a long-lasting manner.

In the text hereinbelow, the term "at least one" is equivalent to the term "one or more".

Preferably, the composition according to the invention comprises neither a colouring agent nor a reducing agent.

According to the present invention, the term "colouring agents" means agents for colouring keratin fibres such as direct dyes, pigments or oxidation dye precursors (bases and couplers). If they are present, their content does not exceed 0.001% by weight relative to the total weight of the composition. Indeed, at such a content, only the composition would be dyed, i.e. no dyeing effect would be observed on the keratin fibres.

It is recalled that oxidation dye precursors, oxidation bases and couplers are colourless or sparingly coloured compounds, which, via a condensation reaction in the presence of an oxidizing agent, give a coloured species. With regard to direct dyes, these compounds are coloured and have a certain affinity for keratin fibres.

According to the present invention, the term "reducing agent" means an agent that is capable of reducing the disulfide bonds of the hair, such as compounds chosen from thiols, alkali metal sulfites, hydrides and phosphines.

In the present invention, the dicarbonyl compounds of formula (I) may be in free form, but also in their hydrate forms or in the form of their salts, preferably in free form or in the form of hydrates.
The salts may be salts derived from the interaction of the compounds of formula (I) with acids or bases, it being possible for the acids or bases to be of organic or mineral nature.

Preferably, the salts are salts derived from the interaction of the compounds of formula (I) with bases. Mention will be made in particular of the salts of alkali metals or alkaline-earth metals and in particular the sodium salts.

Preferably, the dicarbonyl compound(s) corresponding to formula (I) and/or hydrates thereof and/or salts thereof are chosen from the dicarbonyl compounds corresponding to formula (I) in which R represents i) a hydrogen atom or ii) a linear or branched C₁-C₆ alkyl group optionally substituted with a carboxyl group.

More preferably, they are chosen from glyoxylic acid and pyruvic acid and hydrates thereof or salts thereof and more preferentially from glyoxylic acid and the hydrate forms of this compound.

Mention may be made of glyoxylic acid and also the hydrate form thereof (HO)₂CH-C(O)-OH, for instance the glyoxylic acid as a 50% aqueous solution sold by the company Merck.

According to a particularly preferred embodiment, the compound of formula (I) is glyoxylic acid in hydrate form.

According to one embodiment, the composition of the invention comprises from 5% to 15% of one or more dicarbonyl compounds corresponding to formula (I) and/or of hydrate forms thereof and/or salts thereof, preferably from 5% to 10% by weight relative to the total weight of the composition.

As indicated above, the composition according to the invention comprises at least one cationic surfactant.

The term "cationic surfactant" means a surfactant that is positively charged when it is contained in the composition according to the invention. This surfactant may bear one or more permanent positive charges or may contain one or more functions that are cationizable within the composition according to the invention.

The cationic surfactant(s) are preferably chosen from optionally polyoxyalkylenated primary, secondary or tertiary fatty amines, or salts thereof, quaternary ammonium salts, and mixtures thereof.

The fatty amines generally comprise at least one C₈-C₃₀ hydrocarbon-based chain. Among the fatty amines that may be used according to the invention, examples that may be mentioned include stearylamidopropyldimethylamine and distearylamine.
Examples of quaternary ammonium salts that may especially be mentioned include:
- those corresponding to the general formula (II) below: 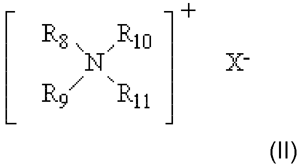 in which the groups R₈ to R₁₁, which may be identical or different, represent a linear or branched aliphatic group comprising from 1 to 30 carbon atoms or an aromatic group such as aryl or alkylaryl, at least one of the groups R₈ to R₁₁ denoting a group comprising from 8 to 30 carbon atoms and preferably from 12 to 24 carbon atoms. The aliphatic groups may comprise heteroatoms especially such as oxygen, nitrogen, sulfur and halogens. The aliphatic groups are chosen, for example, from the groups C₁₋₃₀ alkyl, C₁₋₃₀ alkoxy, polyoxyalkylene (C₂-C₆), C₁₋₃₀ alkylamide, (C₁₂-C₂₂)alkylamido(C₂-C₆)alkyl, (C₁₂-C₂₂)alkylacetate, and C₁₋₃₀ hydroxyalkyl; X⁻ is an anion chosen from the group of halides, phosphates, acetates, lactates, (C₁-C₄)alkyl sulfates and (C₁-C₄)alkyl- or (C₁-C₄)alkylarylsulfonates.
   Among the quaternary ammonium salts of formula (II), preference is given, on the one hand, to tetraalkylammonium salts, for instance dialkyldimethylammonium salts or alkyltrimethylammonium salts in which the alkyl group contains approximately 12 to 22 carbon atoms, in particular behenyltrimethylammonium salts, distearyldimethylammonium salts, cetyltrimethylammonium salts and benzyldimethylstearylammonium salts, or else, on the other hand, to palmitylamidopropyltrimethylammonium salt, stearamidopropyltrimethylammonium salt, stearamidopropyldimethylcetearylammonium salt or the stearamidopropyldimethyl(myristyl acetate)ammonium salt sold under the name Ceraphyl® 70 by the company Van Dyk. It is particularly preferred to use the chloride salts of these compounds.
- quaternary ammonium salts of imidazoline, for instance those of formula (III) below: 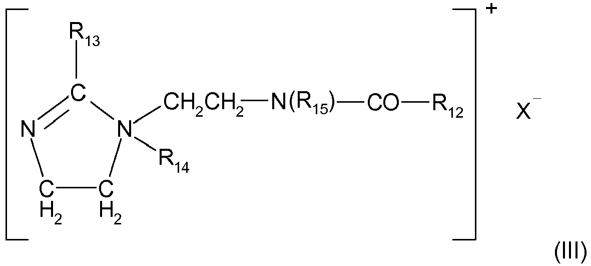 in which R₁₂ represents an alkyl or alkenyl group comprising from 8 to 30 carbon atoms, for example fatty acid derivatives of tallow, R₁₃ represents a hydrogen atom, a C₁-C₄ alkyl group or an alkyl or alkenyl group comprising from 8 to 30 carbon atoms, R₁₄ represents a C₁-C₄ alkyl group, R₁₅ represents a hydrogen atom or a C₁-C₄ alkyl group, X- is an anion chosen from the group of halides, phosphates, acetates, lactates, alkyl sulfates and alkyl-or alkylarylsulfonates, the alkyl and aryl groups of which preferably comprise from 1 to 20 carbon atoms and from 6 to 30 carbon atoms respectively. Preferably, R₁₂ and R₁₃ denote a mixture of alkenyl or alkyl groups comprising from 12 to 21 carbon atoms, for example fatty acid derivatives of tallow, R₁₄ denotes a methyl group and R₁₅ denotes a hydrogen atom. Such a product is sold, for example, under the name Rewoquat® W 75 by the company Rewo;
- quaternary di- or triammonium salts in particular of formula (IV): 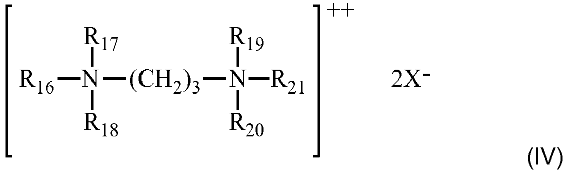 in which R₁₆ denotes an alkyl radical comprising approximately 16 to 30 carbon atoms, optionally hydroxylated and/or interrupted with one or more oxygen atoms, R₁₇ is chosen from hydrogen, an alkyl radical comprising from 1 to 4 carbon atoms and a group [(R₁₆ₐ)(R₁₇ₐ)(R₁₈ₐ)N-(CH₂)₃, R₁₆ₐ, R₁₇ₐ, R₁₈ₐ, R₁₈, R₁₉, R₂₀ and R₂₁, which may be identical or different, are chosen from hydrogen and an alkyl radical comprising from 1 to 4 carbon atoms, and X⁻ is an anion chosen from the group of halides, acetates, phosphates, nitrates and methylsulfates. Such compounds are, for example, Finquat CT-P sold by the company Finetex (Quaternium 89) and Finquat CT sold by the company Finetex (Quaternium 75),
- quaternary ammonium salts containing at least one ester function, such as those of formula (V) below: 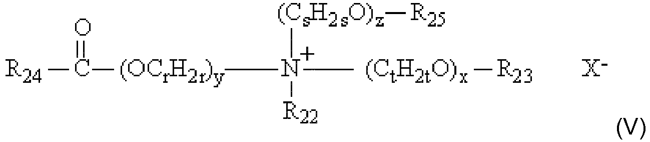 in which:
   R₂₂ is chosen from C₁-C₆ alkyl and C₁-C₆ hydroxyalkyl or dihydroxyalkyl groups;
   R₂₃ is chosen from:
- the group 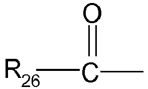
- the groups R₂₇, which are linear or branched, saturated or unsaturated C₁-C₂₂ hydrocarbon-based groups,
- a hydrogen atom,
   R₂₅ is chosen from:
- the group 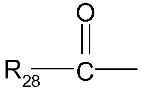
- the groups R₂₉, which are linear or branched, saturated or unsaturated C₁-C₆ hydrocarbon-based groups,
- a hydrogen atom,
   R₂₄, R₂₆ and R₂₈, which may be identical or different, are chosen from linear or branched, saturated or unsaturated C₇-C₂₁ hydrocarbon-based groups;
   r, s and t, which may be identical or different, are integers ranging from 2 to 6;
   r₁ and t₁, which may be identical or different, are equal to 0 or 1;
   and r₂+r₁=2r and t₁+t₂=2t;
   y is an integer ranging from 1 to 10;
   x and z, which may be identical or different, are integers ranging from 0 to 10;
   X- is a simple or complex, organic or mineral anion;
   with the proviso that the sum x + y + z is from 1 to 15, that when x is 0, then R₂₃ denotes R₂₇ and that when z is 0, then R₂₅ denotes R₂₉.

The alkyl groups R₂₂ may be linear or branched, and more particularly linear.
R₂₂ preferably denotes a methyl, ethyl, hydroxyethyl or dihydroxypropyl group, and more particularly a methyl or ethyl group.
Advantageously, the sum x + y + z is from 1 to 10.
When R₂₃ is a hydrocarbon-based group R₂₇, it may be long and may contain from 12 to 22 carbon atoms, or may be short and may contain from 1 to 3 carbon atoms.
When R₂₅ is a hydrocarbon-based group R₂₉, it preferably contains 1 to 3 carbon atoms.
Advantageously, R₂₄, R₂₆ and R₂₈, which may be identical or different, are chosen from linear or branched, saturated or unsaturated C₁₁-C₂₁, hydrocarbon-based groups, and more particularly from linear or branched, saturated or unsaturated C₁₁-C₂₁ alkyl and alkenyl groups.
Preferably, x and z, which may be identical or different, are equal to 0 or 1.
Advantageously, y is equal to 1.
Preferably, r, s and t, which may be identical or different, are equal to 2 or 3, and even more particularly are equal to 2.
The anion X- is preferably a halide (chloride, bromide or iodide) or a alkyl sulfate, more particularly methyl sulfate. However, methanesulfonate, phosphate, nitrate, tosylate, an anion derived from an organic acid, such as acetate or lactate, or any other anion that is compatible with the ammonium containing an ester function, may be used.
The anion X- is even more particularly chloride or methyl sulfate.
Use is made more particularly in the composition according to the invention of the ammonium salts of formula (V) in which:
R₂₂ denotes a methyl or ethyl group,
x and y are equal to 1;
z is equal to 0 or 1;
r, s and t are equal to 2;
R₂₃ is chosen from:
   - the group 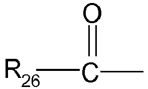
   - methyl, ethyl or C₁₄-C₂₂ hydrocarbon-based groups,
   - a hydrogen atom;
R₂₅ is chosen from:
   - the group 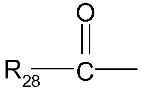
   - a hydrogen atom;
R₂₄, R₂₆ and R₂₈, which may be identical or different, are chosen from linear or branched, saturated or unsaturated C₁₃-C₁₇ hydrocarbon-based groups and preferably from linear or branched, saturated or unsaturated C₁₃-C₁₇ alkyl and alkenyl groups.
The hydrocarbon-based groups are advantageously linear.

Examples that may be mentioned include the compounds of formula (V) such as the diacyloxyethyldimethylammonium, diacyloxyethylhydroxyethylmethylammonium, monoacyloxyethyldihydroxyethylmethylammonium, triacyloxyethylmethylammonium and monoacyloxyethylhydroxyethyldimethylammonium salts (chloride or methyl sulfate in particular), and mixtures thereof. The acyl groups preferably contain 14 to 18 carbon atoms and are obtained more particularly from a plant oil such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with C₁₀-C₃₀ fatty acids or with mixtures of C₁₀-C₃₀ fatty acids of plant or animal origin, or by transesterification of the methyl esters thereof. This esterification is followed by a quaternization using an alkylating agent such as an alkyl halide (preferably a methyl or ethyl halide), a dialkyl sulfate (preferably dimethyl or diethyl sulfate), methyl methanesulfonate, methyl paratoluenesulfonate, glycol chlorohydrin or glycerol chlorohydrin.
Such compounds are sold, for example, under the names Dehyquart® by the company Henkel, Stepanquat® by the company Stepan, Noxamium® by the company CECA or Rewoquat® WE 18 by the company Rewo-Witco.
The composition according to the invention may contain, for example, a mixture of quaternary ammonium salts of mono-, di- and triesters with a weight majority of diester salts.
It is also possible to use the ammonium salts containing at least one ester function that are described in patents US-A-4 874 554 and US-A-4 137 180.
Use may be made of behenoylhydroxypropyltrimethylammonium chloride sold by KAO under the name Quatarmin BTC 131.
Preferably, the ammonium salts containing at least one ester function contain two ester functions.
Among the quaternary ammonium salts containing at least one ester function that may be used, dipalmitoylethylhydroxyethylmethylammonium salts are preferably used.

Preferably, the cationic surfactant(s) are chosen from cetyltrimethylammonium, behenyltrimethylammonium and dipalmitoylethylhydroxyethylmethylammonium salts, and mixtures thereof, and more particularly behenyltrimethylammonium chloride, cetyltrimethylammonium chloride, and dipalmitoylethylhydroxyethylammonium methosulfate, and mixtures thereof.

The composition according to the invention preferably comprises the cationic surfactant(s) in an amount ranging from 0.1% to 15% by weight, better still from 0.2% to 8% by weight and even more preferentially from 0.4% to 3% by weight relative to the total weight of the composition.

The weight ratio of dicarbonyl compounds corresponding to formula (I) and/or hydrate forms thereof and/or salts thereof/cationic surfactants may range from 0.1 to 20 and better still from 1 to 10.

The composition according to the invention also comprises one or more non liquid fatty substances. Optionally the composition may also contain one or more liquid fatty substances.

The term "*fatty substance*" means an organic compound that is insoluble in water at ordinary temperature (25°C) and at atmospheric pressure (760 mmHg) (solubility of less than 5%, preferably 1% and even more preferentially 0.1%). They have in their structure at least one hydrocarbon-based chain containing at least 6 carbon atoms or a sequence of at least two siloxane groups. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

The fatty substances of the invention do not contain any salified carboxylic acid groups.

In particularly, the fatty substances that are useful in the composition of the invention are not (poly)oxyalkylenated or (poly)glycerolated ethers.

According to the invention, the fatty substance of the invention is a compound that is non-liquid at a temperature of 25°C and at atmospheric pressure.

The term "non-liquid" preferably refers to a solid compound or a compound with a viscosity of greater than 2 Pa.s at a temperature of 25°C and at a shear rate of 1 s⁻¹.

The term "*oil*" means a "*fatty substance*" which is liquid at room temperature (25°C) and at atmospheric pressure (760 mmHg).

The term "*non-silicone oil*" means an oil not containing any silicon atoms (Si) and the term "*silicone oil*" means an oil containing at least one silicon atom.

More particularly, the fatty substances (liquid and non liquid) are chosen from C₆-C₁₆ hydrocarbons, hydrocarbons containing more than 16 carbon atoms, non-silicone oils of animal origin, plant oils of triglyceride type, synthetic triglycerides, fluoro oils, fatty alcohols, esters of fatty acids and/or of fatty alcohols other than triglycerides, and plant waxes, non-silicone waxes and silicones, and mixtures thereof.

It is recalled that the fatty alcohols, fatty esters and fatty acids more particularly contain one or more linear or branched, saturated or unsaturated hydrocarbon-based groups comprising 6 to 30 carbon atoms, better still 8 to 30 carbon atoms, which are optionally substituted, in particular with one or more (in particular 1 to 4) hydroxyl groups. If they are unsaturated, these compounds may comprise one to three conjugated or unconjugated carbon-carbon double bonds.

As regards the C₆-C₁₆ hydrocarbons, they are linear or branched, and optionally cyclic, and are preferably alkanes. Examples that may be mentioned include hexane, dodecane and isoparaffins such as isohexadecane and isodecane.

A hydrocarbon-based oil of animal origin that may be mentioned is perhydrosqualene.

The triglyceride oils of plant or synthetic origin are preferably chosen from liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, palm oil, caprylic/capric acid triglycerides, for instance those sold by the company Stéarineries Dubois or those sold under the names Miglyol^{®} 810, 812 and 818 by the company Dynamit Nobel, jojoba oil and shea butter oil.

The linear or branched hydrocarbons of mineral or synthetic origin containing more than 16 carbon atoms are preferably chosen from liquid paraffins, petroleum jelly, liquid petroleum jelly, polydecenes, and hydrogenated polyisobutene such as Parleam®.

As regards the C₆-C₁₆ alkanes, they are linear or branched, and optionally cyclic. Examples that may be mentioned include hexane, dodecane and isoparaffins such as isohexadecane and isodecane.

As oils of animal, plant, mineral or synthetic origin that may be used in the composition of the invention, examples that may be mentioned include:
the fluoro oils may be chosen from perfluoromethylcyclopentane and perfluoro-1,3-dimethylcyclohexane, sold under the names Flutec® PC1 and Flutec® PC3 by the company BNFL Fluorochemicals; perfluoro-1,2-dimethylcyclobutane; perfluoroalkanes such as dodecafluoropentane and tetradecafluorohexane, sold under the names PF 5050® and PF 5060® by the company 3M, or bromoperfluorooctyl sold under the name Foralkyl® by the company Atochem; nonafluoromethoxybutane and nonafluoroethoxyisobutane; perfluoromorpholine derivatives such as 4-trifluoromethylperfluoromorpholine sold under the name PF 5052® by the company 3M.

The fatty alcohols which are suitable for the implementation of the invention are more particularly chosen from saturated or unsaturated and linear or branched alcohols comprising from 6 to 30 carbon atoms and preferably from 8 to 30 carbon atoms. The fatty alcohols of the invention are in particular of formula R-OH, R denoting a linear or branched alkyl or alkenyl radical comprising from 6 to 30 carbon atoms and preferably from 8 to 30 carbon atoms. Mention may be made, for example, of cetyl alcohol, stearyl alcohol and a mixture thereof (cetylstearyl alcohol), octyldodecanol, 2-butyloctanol, 2-hexyldecanol, 2-undecylpentadecanol, oleyl alcohol or linoleyl alcohol.

Preferably, the non liquid fatty alcohol is linear and saturated, i.e. R denotes a linear alkyl radical containing from 6 to 30 carbon atoms and preferably from 8 to 30 carbon atoms.

As regards the esters of fatty acid and/or of fatty alcohols, advantageously other than the triglycerides mentioned previously, mention may be made especially of esters of saturated or unsaturated, linear or branched C₁-C₂₆ aliphatic mono- or polyacids and of saturated or unsaturated, linear or branched C₁-C₂₆ aliphatic mono- or polyalcohols, the total carbon number of the esters being greater than or equal to 6 and more advantageously greater than or equal to 10.
Among the monoesters, mention may be made of dihydroabietyl behenate; octyldodecyl behenate; isocetyl behenate; cetyl lactate; C₁₂-C₁₅ alkyl lactate; isostearyl lactate; lauryl lactate; linoleyl lactate; oleyl lactate; (iso)stearyl octanoate; isocetyl octanoate; octyl octanoate; cetyl octanoate; decyl oleate; isocetyl isostearate; isocetyl laurate; isocetyl stearate; isodecyl octanoate; isodecyl oleate; isononyl isononanoate; isostearyl palmitate; methylacetyl ricinoleate; myristyl stearate; octyl isononanoate; 2-ethylhexyl isononanoate; octyl palmitate; octyl pelargonate; octyl stearate; octyldodecyl erucate; oleyl erucate; ethyl and isopropyl palmitates, 2-ethylhexyl palmitate, 2-octyldecyl palmitate, alkyl myristates such as isopropyl, butyl, cetyl, 2-octyldodecyl, myristyl or stearyl myristate, hexyl stearate, butyl stearate, isobutyl stearate; dioctyl malate, hexyl laurate, 2-hexyldecyl laurate.
Still within the context of this variant, it is also possible to use esters of C₄-C₂₂ dicarboxylic or tricarboxylic acids and of C₁-C₂₂ alcohols, and esters of monocarboxylic, dicarboxylic or tricarboxylic acids and of C₂-C₂₆ di-, tri-, tetra- or pentahydroxy alcohols.

Mention may in particular be made of: diethyl sebacate; diisopropyl sebacate; diisopropyl adipate; di(n-propyl) adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol distearates.

Among the esters mentioned above, use is preferably made of ethyl, isopropyl, myristyl, cetyl or stearyl palmitate, 2-ethylhexyl palmitate, 2-octyldecyl palmitate, alkyl myristates, such as isopropyl, butyl, cetyl or 2-octyldodecyl myristate, hexyl stearate, butyl stearate, isobutyl stearate, dioctyl malate, hexyl laurate, 2-hexyldecyl laurate, isononyl isononanoate or cetyl octanoate.
Preferably among the esters mentioned above, non liquid esters are selected from myristyl, cetyl or stearyl palmitate.

The composition may also comprise, as fatty ester, sugar esters and diesters of C₆-C₃₀ and preferably C₁₂-C₂₂ fatty acids. It is recalled that the term "*sugar*" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Mention may be made, as suitable sugars, for example, of sucrose (or saccharose), glucose, galactose, ribose, fucose, maltose, fructose, mannose, arabinose, xylose and lactose, and derivatives thereof, in particular alkyl derivatives, such as methyl derivatives, for instance methylglucose.

The sugar esters of fatty acids may be selected especially from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise from one to three conjugated or unconjugated carbon-carbon double bonds.

The esters according to this alternative form may also be chosen from mono-, di-, tri- and tetraesters, polyesters, and mixtures thereof.

These esters may be, for example, oleates, laurates, palmitates, myristates, behenates, cocoates, stearates, linoleates, linolenates, caprates and arachidonates, or mixtures thereof such as, especially, oleopalmitate, oleostearate and palmitostearate mixed esters.

More particularly, use is made of monoesters and diesters and in particular mono- or dioleate, -stearate, -behenate, -oleate/palmitate, -linoleate, -linolenate or -oleate/stearate of sucrose, of glucose or of methylglucose.

An example that may be mentioned is the product sold under the name Glucate® DO by the company Amerchol, which is a methylglucose dioleate.

Examples of esters or mixtures of esters of sugar and of fatty acid that may also be mentioned include:
- the products sold under the names F160, F140, F110, F90, F70 and SL40 by the company Crodesta, respectively denoting sucrose palmitostearates formed from 73% monoester and 27% diester and triester, from 61% monoester and 39% diester, triester and tetraester, from 52% monoester and 48% diester, triester and tetraester, from 45% monoester and 55% diester, triester and tetraester, from 39% monoester and 61% diester, triester and tetraester, and sucrose monolaurate;
- the products sold under the name Ryoto Sugar Esters, for example referenced B370 and corresponding to sucrose behenate formed from 20% monoester and 80% diester-triester-polyester;
- the sucrose monopalmitate/stearate-dipalmitate/stearate sold by the company Goldschmidt under the name Tegosoft® PSE.

The non-silicone wax(es) are chosen in particular from carnauba wax, candelilla wax, esparto wax, paraffin wax, ozokerite, plant waxes, such as olive tree wax, rice wax, hydrogenated jojoba wax or absolute flower waxes, such as the blackcurrant blossom essential wax sold by Bertin (France), or animal waxes, such as beeswaxes or modified beeswaxes (cerabellina); other waxes or waxy starting materials which can be used according to the invention are in particular marine waxes, such as that sold by Sophim under the reference M82, polyethylene waxes or polyolefin waxes in general.

The silicones that may be used in the colouring composition of the present invention are volatile or non-volatile, cyclic, linear or branched silicones, which are unmodified or modified with organic groups, having a viscosity from 5×10⁻⁶ to 2.5 m²/s at 25°C, and preferably 1×10⁻⁵ to 1 m²/s.

The silicones that may be used in accordance with the invention may be in the form of oils, waxes, resins or gums.

Preferably, the silicone is chosen from polydialkylsiloxanes, especially polydimethylsiloxanes (PDMS), and organomodified polysiloxanes comprising at least one functional group chosen from amino groups and alkoxy groups.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and more particularly still from:
(i) cyclic polydialkylsiloxanes containing from 3 to 7 and preferably from 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, and Silbione^{®} 70045 V5 by Rhodia, and mixtures thereof.

Mention may also be made of cyclocopolymers of the dimethylsiloxane/methylalkylsiloxane type, such as Volatile Silicone® FZ 3109 sold by the company Union Carbide, of formula:

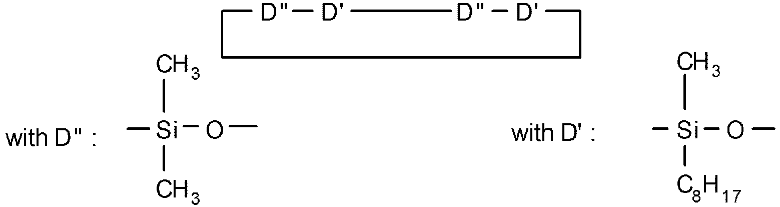

Mention may also be made of mixtures of cyclic polydialkylsiloxanes with organosilicon compounds, such as the mixture of octamethylcyclotetrasiloxane and tetra(trimethylsilyl)pentaerythritol (50/50) and the mixture of octamethylcyclotetrasiloxane and oxy-1,1'-bis(2,2,2',2',3,3'-hexatrimethylsilyloxy)neopentane;
(ii) linear volatile polydialkylsiloxanes containing 2 to 9 silicon atoms and having a viscosity of less than or equal to 5×10⁻⁶ m²/s at 25°C. An example is
   decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics.

Use is preferably made of non-volatile polydialkylsiloxanes, polydialkylsiloxane gums and resins, polyorganosiloxanes modified with the organofunctional groups above, and mixtures thereof.

These silicones are more particularly chosen from polydialkylsiloxanes, among which mention may be made mainly of polydimethylsiloxanes having trimethylsilyl end groups. The viscosity of the silicones is measured at 25°C according to ASTM standard 445 Appendix C.
Mention may be made, among these polydialkylsiloxanes, without implied limitation, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by Rhodia;
- the oils of the 200 series from the company Dow Corning, such as DC200 with a viscosity of 60 000 mm²/s;
- the Viscasil^{®} oils from General Electric and certain oils of the SF series (SF 96, SF 18) from General Electric.

Mention may also be made of polydimethylsiloxanes bearing dimethylsilanol end groups known under the name dimethiconol (CTFA), such as the oils of series 48 from the company Rhodia.

In this category of polydialkylsiloxanes, mention may also be made of the products sold under the names Abil Wax® 9800 and 9801 by the company Goldschmidt, which are polydi(C₁-C₂₀)alkylsiloxanes.

The silicone gums that may be used in accordance with the invention are in particular polydialkylsiloxanes and preferably polydimethylsiloxanes with high number-average molecular weights of between 200 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane, tridecane or their mixtures.

Products which can be used more particularly in accordance with the invention are mixtures such as:
- the mixtures formed from a polydimethylsiloxane hydroxylated at the chain end, or dimethiconol (CTFA), and from a cyclic polydimethylsiloxane, also known as cyclomethicone (CTFA), such as the product Q2 1401 sold by Dow Corning;
- the mixtures of a polydimethylsiloxane gum and of a cyclic silicone, such as the product SF 1214 Silicone Fluid from General Electric; this product is an SF 30 gum corresponding to a dimethicone, having a number-average molecular weight of 500 000, dissolved in the oil SF 1202 Silicone Fluid corresponding to decamethylcyclopentasiloxane;
- the mixtures of two PDMSs with different viscosities, and more particularly of a PDMS gum and a PDMS oil, such as the product SF 1236 from General Electric. The product SF 1236 is a mixture of a gum SE 30 defined above, with a viscosity of 20 m²/s, and of an oil SF 96 with a viscosity of 5×10⁻⁶ m²/s. This product preferably comprises 15% of gum SE 30 and 85% of an oil SF 96.
The organopolysiloxane resins that may be used in accordance with the invention are crosslinked siloxane systems containing the following units:

R₂SiO_{2/2}, R₃SiO_{1/2}, RSiO_{3/2} and SiO_{4/2},

in which R represents an alkyl containing 1 to 16 carbon atoms. Among these products, those that are particularly preferred are those in which R denotes a C₁-C₄ lower alkyl group, more particularly methyl.

Mention may be made, among these resins, of the product sold under the name Dow Corning 593 or those sold under the names Silicone Fluid SS 4230 and SS 4267 by General Electric, which are silicones of dimethyl/trimethylsiloxane structure.
Mention may also be made of the resins of the trimethylsiloxysilicate type, sold especially under the names X22-4914, X21-5034 and X21-5037 by the company Shin-Etsu.
The organomodified silicones that may be used in accordance with the invention are silicones as defined above and comprising in their structure one or more organofunctional groups attached via a hydrocarbon-based group.

In addition to the silicones described above, the organomodified silicones can be polydiarylsiloxanes, in particular polydiphenylsiloxanes, and polyalkylarylsiloxanes functionalized by the abovementioned organofunctional groups.

The polyalkylarylsiloxanes are chosen particularly from linear and/or branched polydimethyl/methylphenylsiloxanes and polydimethyl/diphenylsiloxanes with a viscosity of from 1×10⁻⁵ to 5×10⁻² m²/s at 25°C.

Among these polyalkylarylsiloxanes, examples that may be mentioned include the products sold under the following names:
▪ the Silbione^{®} oils of the 70 641 series from Rhodia;
▪ the oils of the Rhodorsil^{®} 70 633 and 763 series from Rhodia;
▪ the oil Dow Corning 556 Cosmetic Grade Fluid from Dow Corning;
▪ the silicones of the PK series from Bayer, such as the product PK20;
▪ the silicones of the PN and PH series from Bayer, such as the products PN1000 and PH1000;
. certain oils of the SF series from General Electric, such as SF 1023, SF 1154, SF 1250 and SF 1265.

Mention may be made, among the organomodified silicones, of polyorganosiloxanes comprising:
- substituted or unsubstituted amine groups, such as the products sold under the name GP 4 Silicone Fluid and GP 7100 by the company Genesee, or the products sold under the names Q2 8220 and Dow Corning 929 or 939 by the company Dow Corning. The substituted amine groups are, in particular, C₁-C₄ aminoalkyl groups;
- alkoxylated groups, such as the product sold under the name Silicone Copolymer F-755 by SWS Silicones, and Abil Wax® 2428, 2434 and 2440 by the company Goldschmidt.

More particularly, the non-liquid fatty substances are chosen from fatty alcohols, esters of a fatty acid and/or of a fatty alcohol, non-silicone waxes, silicones and fatty ethers, which are non-liquid and preferably solid.

Preferably, the fatty substance is a non-silicone compound. The term "non-silicone fatty substance" means a fatty substance whose structure does not comprise any silicon atoms.

More preferably, the fatty substances are chosen from fatty alcohols and in particular fatty alcohols that are non-liquid and preferably solid.

The fatty substance(s) as described above are preferably present in an amount ranging from 1% to 20% by weight and even better still from 5% to 20% by weight relative to the total weight of the composition.

Preferably, the non liquid fatty substance(s) as described above are preferably present in an amount ranging from 1% to 20% by weight and even better still from 5% to 20% by weight relative to the total weight of the composition.

According to a particular embodiment, the composition comprises at least one amphoteric or zwitterionic surfactant.

In particular, the amphoteric or zwitterionic surfactant(s), which are preferably non-silicone, which may be used in the present invention may especially be derivatives of optionally quaternized aliphatic secondary or tertiary amines, in which derivatives the aliphatic group is a linear or branched chain comprising from 8 to 22 carbon atoms, the said amine derivatives containing at least one anionic group, for instance a carboxylate, sulfonate, sulfate, phosphate or phosphonate group.

Mention may be made in particular of (C₈-C₂₀)alkylbetaines, (C₈-C₂₀)alkylsulfobetaines, (C₈-C₂₀)alkylamido(C₃-C₈)alkylbetaines and (C₈-C₂₀)alkylamido(C₆-C₈)alkylsulfobetaines.

Among the optionally quaternized secondary or tertiary aliphatic amine derivatives that may be used, as defined above, mention may also be made of the compounds of respective structures **(B1)** and **(B2)** below:

Rₐ-C(O)-NH-CH₂-CH₂-N⁺(R_{b})(R_{c})-CH₂C(O)O⁻, M⁺, X⁻ **(B1)**

in which formula:
▪ Rₐ represents a C₁₀-C₃₀ alkyl or alkenyl group derived from an acid Rₐ-COOH preferably present in hydrolysed coconut oil, or a heptyl, nonyl or undecyl group;
▪ R_{b} represents a β-hydroxyethyl group; and
▪ R_{c} represents a carboxymethyl group;
▪ M⁺ represents a cationic counterion derived from an alkali metal or alkaline-earth metal, such as sodium, an ammonium ion or an ion derived from an organic amine, and
▪ X⁻ represents an organic or mineral anionic counterion, such as that chosen from halides, acetates, phosphates, nitrates, (C₁-C₄)alkyl sulfates, (C₁-C₄)alkyl-or (C₁-C₄)alkylarylsulfonates, in particular methyl sulfate and ethyl sulfate; or alternatively M⁺ and X⁻ are absent;

R_{a'}-C(O)-NH-CH₂-CH₂-N(B)(B') **(B2)**

in which formula:
▪ B represents the group -CH₂-CH₂-O-X';
▪ B' represents the group -(CH₂)_{z}Y', with z = 1 or 2;
▪ X' represents the group -CH₂-C(O)OH, -CH₂-C(O)OZ', -CH₂-CH₂-C(O)OH, -CH₂-CH₂-C(O)OZ', or a hydrogen atom;
▪ Y' represents the group -C(O)OH, -C(O)OZ', -CH₂-CH(OH)-SO₃H or the group -CH₂-CH(OH)-SO₃-Z';
▪ Z' represents a cationic counterion derived from an alkali metal or alkaline-earth metal, such as sodium, an ammonium ion or an ion derived from an organic amine;
▪ R_{a'} represents a C₁₀-C₃₀ alkyl or alkenyl group of an acid R_{a'}-C(O)OH preferably present in hydrolysed linseed oil or coconut oil, an alkyl group, especially of C₁₇ and its iso form, or an unsaturated C₁₇ group.

The compounds of this type are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol^{®} C2M Concentrate.

Use may also be made of compounds of formula (B'2):

**Ra"-NH-CH(Y")-(CH₂)n-C(O)-NH-(CH₂)n'-N(Rd)(Re)** **(B'2)**

in which formula:
▪ Y" represents the group -C(O)OH, -C(O)OZ", -CH₂-CH(OH)-SO₃H or the group -CH₂-CH(OH)-SO₃-Z";
▪ Rd and Re, independently of each other, represent a C₁-C₄ alkyl or hydroxyalkyl radical;
▪ Z" represents a cationic counterion derived from an alkali metal or alkaline-earth metal, such as sodium, an ammonium ion or an ion derived from an organic amine;
▪ Ra" represents a C₁₀-C₃₀ alkyl or alkenyl group of an acid Ra"-C(O)OH preferably present in coconut oil or in hydrolysed linseed oil;
▪ n and n' denote, independently of each other, an integer ranging from 1 to 3.

Among the compounds of formula (B'2), mention may be made of the compound classified in the CTFA dictionary under the name sodium diethylaminopropyl cocoaspartamide and sold by the company Chimex under the name Chimexane HB.

In accordance with a particular embodiment of the invention, the content of amphoteric or zwitterionic surfactant(s), when they are present, ranges from 0.05% to 30% by weight, preferably from 0.5% to 10% by weight and more preferably from 0.1 % to 5% by weight, relative to the total weight of the composition.

The composition according to the invention may also comprise a cellulose-based polymer.

According to the invention, the term "cellulose-based" polymer means any polysaccharide compound having in its structure sequences of glucose residues linked together via β-1,4 bonds; in addition to unsubstituted celluloses, the cellulose derivatives may be anionic, cationic, amphoteric or nonionic. Thus, the cellulose polymers of the invention may be chosen from unsubstituted celluloses, including those in a microcrystalline form, and cellulose ethers. Among these cellulose polymers, cellulose ethers, cellulose esters and cellulose ester ethers are distinguished. Among the cellulose esters are mineral cellulose esters (cellulose nitrates, sulfates, phosphates, etc.), organic cellulose esters (cellulose monoacetates, triacetates, amidopropionates, acetatebutyrates, acetatepropionates and acetatetrimellitates, etc.), and mixed organic/mineral cellulose esters, such as cellulose acetatebutyrate sulfates and cellulose acetatepropionate sulfates. Among the cellulose ester ethers, mention may be made of hydroxypropylmethylcellulose phthalates and ethylcellulose sulfates.

The compositions according to the invention may be provided in any galenical form conventionally used and in particular in the form of an aqueous, alcoholic or aqueous/alcoholic solution or suspension or oily solution or suspension; a solution or a dispersion of the lotion or serum type; an emulsion, in particular of liquid or semi-liquid consistency, of the O/W, W/O or multiple type; a suspension or emulsion of soft consistency of (O/W) or (W/O) cream type; an aqueous or anhydrous gel, or any other cosmetic form.

These compositions may be packaged in pump-action bottles or in aerosol containers, so as to apply the composition in vaporized (lacquer) form or in the form of a mousse. Such packaging forms are indicated, for example, when it is desired to obtain a spray or a mousse, for the treatment of the hair. In these cases, the composition preferably comprises at least one propellant.

The compositions of the invention may be aqueous or anhydrous. They are preferably aqueous and then comprise water at a concentration ranging from 5% to 98%, better still from 5% to 90% and even better still from 10% to 90% by weight relative to the total weight of the composition.

The composition may in particular comprise one or more organic solvents, in particular water-soluble solvents, such as C₁-C₇ alcohols; mention may in particular be made of C₁-C₇ aliphatic monoalcohols, C₆-C₇ aromatic monoalcohols, C₃-C₇ polyols or C₃-C₇ polyol ethers, which may be employed alone or as a mixture with water.

The composition of the invention may also comprise at least one common cosmetic ingredient, chosen in particular from propellants; surfactants other than those described previously; sunscreens; moisturizers; antidandruff agents; antioxidants; chelating agents; nacreous agents and opacifiers; plasticizers or coalescers; fillers; silicones and in particular polydimethylsiloxanes; polymeric or non-polymeric thickeners or gelling agents other than the cellulose-based polymers already mentioned; emulsifiers; polymers, in particular conditioning or styling polymers; fragrances; basifying agents such as sodium hydroxide or acidifying agents; silanes; crosslinking agents. Needless to say, the composition may comprise several cosmetic ingredients featured in the above list.

Depending on their nature and the intended use of the composition, the common cosmetic ingredients may be present in usual amounts which may be readily determined by a person skilled in the art and which may be, for each ingredient, between 0.01% and 80% by weight. A person skilled in the art will take care to choose the ingredients included in the composition, and also the amounts thereof, such that they do not harm the properties of the compositions of the present invention.

The pH of the composition is less than or equal to 4, and preferably ranges from 1 to 4, even more preferentially from 1 to 3, better still from 1.5 to 3 and even better still from 1.7 to 3.

It may be adjusted to the desired value by means of acidifying and/or basifying agents usually used for treating keratin fibres.

The basifying agent may be chosen from mineral or organic or hybrid alkaline agents, or mixtures thereof.

The mineral alkaline agent(s) are preferably chosen from aqueous ammonia, alkali metal carbonates or bicarbonates such as sodium or potassium carbonate and sodium or potassium bicarbonate, sodium hydroxide or potassium hydroxide, or mixtures thereof.

The organic alkaline agent(s) are preferably chosen from organic amines with a pKb at 25°C of less than 12, preferably less than 10 and even more advantageously less than 6. It should be noted that it is the pKb corresponding to the function of highest basicity.

Mention may be made, as hybrid compounds, of the salts of the abovementioned amines with acids, such as carbonic acid or hydrochloric acid.
The organic alkaline agent(s) are chosen, for example, from amine derivatives such as alkanolamines, oxyethylenated and/or oxypropylenated ethylenediamines, amino acids of amines such as a 1,3-diaminopropane, 1,3-diamino-2-propanol, spermine or spermidine.

The term "alkanolamine" means an organic amine comprising a primary, secondary or tertiary amine function, and one or more linear or branched C₁-C₈ alkyl groups bearing one or more hydroxyl radicals.

Sodium hydroxide is in particular suitable for use in the invention.

The acidifying agent may be chosen from mineral or organic acids, for instance hydrochloric acid, phosphoric acid or lactic acid.

The composition according to the invention is preferably in the form of styling or care gels, care lotions or creams, conditioners, masks or sera.

The composition according to the invention may be obtained by mixing several compositions.

The process of the invention comprises the application of the composition described previously, followed by a step of straightening the hair with an iron, preferably at a temperature of at least 150°C. The straightening iron is known in the prior art. It consists in straightening the hair with flat heating tongs, which are generally metallic. The straightening irons are generally used at a temperature ranging from 150 to 250°C.

The process of the invention may comprise other intermediate steps aimed at improving the straightening of the hair.

According to a particular embodiment, the process of the invention comprises the application of the composition of the invention to dry hair, and a time of contact of the composition with the hair ranging from 10 to 60 minutes and preferably from 20 to 40 minutes. After this leave-on time, straightening with a brush and with a hairdryer (blow-drying) is performed. The hair is then straightened with a straightening iron at a temperature ranging from 150 to 250°C and preferably from 210 to 230°C.

The process of the invention may comprise the application of other hair agents as a pretreatment or post-treatment. In particular, it may comprise the application of a conditioning care product as a post-treatment.

According to another embodiment, the hair straightening process comprises a step of washing the hair and then of drying with a hairdryer before applying the composition of the invention. According to this particular embodiment, the steps described above are then encountered, such as the contact time of the composition, the straightening with a straightening iron, the application of a conditioning agent and the rinsing, all these steps possibly being performed independently of each other, blow-drying possibly being intercalated between the contact of the composition according to the invention and the straightening with the iron. According to a particular embodiment, the straightening with the straightening iron is performed in several passes on the hair, in general 8 to 10 passes.

The process of the present invention is preferably performed without a step of permanent reshaping at basic pH or based on a reducing agent.

### Examples

The following compositions were produced in accordance with the invention.

| | 1 | 2 | 3 | 4 | 5 | 6 | |
|---|---|---|---|---|---|---|---|
| Glyoxylic acid as an aqueous 50% solution | 10 | 10 | 10 | 10 | 10 | 10 | |
| Liquid petroleum jelly (Blandol from Sonneborn) | | 10 | | | | | |
| Myristyl alcohol | | | 0.4 | | | | |
| Cetylstearyl alcohol (50/50 C16/C18) | 5 | 5 | | | 3.5 | 3.5 | |
| Glyceryl esters of isostearic, adipic plant fatty acids (Softisan 649 from Cremer Oleo) | | | | 0.15 | | | |
| Cetyl alcohol | | | 4.5 | 3.5 | | | |
| Myristyl/cetyl/stearyl myristate/palmitate/stearate mixture (Crodamol MS-PA-(MH) from Croda) | 1 | 1 | 1 | 0.25 | | | |
| Palm oil (refined palm oil/SG from SIO) | | | | | 2 | 2 | |
| Cetyltrimethylammonium chloride as an aqueous 25% solution (Genamin CTAC 25 from Clariant) | | | 2.5 | 2 | | | |
| Behenyltrimethylammonium chloride as a 79% solution in water/isopropanol (Genamin KDMP from Clariant) | 1 | 1 | 0.6 | | 2 | 2 | |
| Stearylamidopropyldimethylamine (Mackine 301 from Rhodia) | | | | 0.75 | 0.25 | 0.25 | |
| Dipalmitoylethylhydroxyethylmethylammonium methosulfate (30)/cetearyl alcohol (70) (Dehyquart F 30 from Cognis) | | | 1 | | | | |
| Fragrance | 0.7 | 0.7 | 0.5 | 0.5 | 0.5 | 0.5 | |
| Sodium hydroxide | qs pH 2.2 | qs pH 2.2 | qs pH 2.2 | qs pH 2.2 | qs pH 2.2 | qs pH 3 | |
| Water | qs 100 g | qs 100 g | qs 100 g | qs 100 g | qs 100 g | qs 100 g | |

| | 7 | 8 | 9 | 10 | 11 | 12 | 13 |
|---|---|---|---|---|---|---|---|
| Glyoxylic acid as an aqueous 50% solution | 16 | 16 | 16 | 16 | 16 | 16 | 16 |
| Lactic acid | 2.5 | 2.5 | 2.5 | 2.5 | 2.5 | 2.5 | 2.5 |
| Liquid petroleum jelly (Blandol from Sonneborn) | | | 10 | | | | |
| Myristyl alcohol | | | | 0.4 | 0.4 | | |
| Cetylstearyl alcohol (50/50 C16/C18) | 5 | 5 | 5 | | | | 3 |
| Glyceryl esters of isostearic, adipic plant fatty acids (Softisan 649 from Cremer Oleo) | | | | | | 0.15 | |
| Cetyl alcohol | | | | 4.5 | 4.5 | 3.5 | |
| Myristyl/cetyl/stearyl myristate/palmitate/stearate mixture (Crodamol MS-PA-(MH) from Croda) | 1 | 1 | 1 | 1 | 1 | 0.25 | 0.5 |
| Palm oil (refined palm oil/SG from SIO) | | | | | | | |
| Cetyltrimethylammonium chloride as an aqueous 25% solution (Genamin CTAC 25 from Clariant) | | | | 2.5 | 2.5 | 2 | |
| Behenyltrimethylammonium chloride as a 79% solution in water/isopropanol (Genamin KDMP from Clariant) | 1 | 1 | 1 | 0.6 | 0.6 | | 1.5 |
| Stearylamidopropyldimethylamine (Mackine 301 from Rhodia) | | | | | | 0.75 | |
| Dipalmitoylethylhydroxyethylmethylammonium methosulfate (30)/cetearyl alcohol (70) (Dehyquart F 30 from Cognis) | | | | 1 | 1 | | |
| Fragrance | 0.7 | 0.7 | 0.7 | 0.7 | 0.7 | 0.7 | |
| Sodium hydroxide | qs pH 2.2 | qs pH 2.2 | qs pH 2.2 | qs pH 2.2 | qs pH 2.2 | qs pH 2.2 | qs pH 2.2 |
| Water | qs 100 g | qs 100 g | qs 100 g | qs 100 g | qs 100 g | qs 100 g | qs 100 g |

The compositions according to the invention are stable over time, in a temperature range of from 4°C to 40°C.

The compositions are applied to curly hair, which may be natural or dyed, or sensitized by a prior bleaching step at a rate of 1 g per 2 g of hair. After 15 minutes, the hair is rinsed, dried with a hairdryer (blow-drying) and then straightened by treating with flat tongs heated to 210°C. It is subsequently shampooed to examine the remanence of the straightening effects and of modification of the mechanical and cosmetic properties of the fibres.

During the blow-drying before straightening with flat tongs, a small amount of evolution of fumes is observed.

The compositions according to the invention make it possible to obtain good straightening properties in terms of curl relaxation, protection of the natural or artificial colour, resistance of the fibres to mechanical stresses (pulling, rubbing, twisting), in particular by limiting breakage, sheen, smooth feel and smooth look. These effects are also remanent with respect to hair maintenance products such as shampoos, hair conditioners and hair dyes.

### Comparative example :

Compositions A, B and C were prepared with the following compositions :

| Compositions | A (invention) (% wt/wt) | B (invention) (%wt/wt) | C (comparative) (%wt/wt) |
|---|---|---|---|
| GLYOXYLIC ACID | 5 AM | 5 AM | 5 AM |
| BEHENTRIMONIUM CHLORIDE | 0,79 AM | 0,79 AM | 0,79 AM |
| CETEARYL ALCOHOL | 1.5% AM | 6% AM | 0.25% AM |
| SODIUM HYDROXIDE | QS pH 2.2 | QS pH 2.2 | QS pH 2.2 |
| DEIONIZED WATER | OS 100 | OS 100 | OS 100 |

2.7g hair locks of curl type IV bleached hair were washed with a shampoo and blow dried. Then 2.7g of composition A was applied to one of the hair lock and 2.7g of composition B was applied to another hair lock and 2.7g of composition C was applied to another hair lock. After a leave-on time of 20 minutes on the hair, the locks were blow-dried with a hairdryer (brushing with 15 passes of a brush) and were then straightened with a straightening iron (10 passes). The locks were then washed with a shampoo and let dried naturally (spontaneous).

Qualities of use and conditioning performances were improved by using composition A.
In particular, the flat iron was easily applied on the hair lock treated with composition A and B. Hair was smoother and silkier after being treated by compositions A and B. The hair was more conditioned after being treated by composition B The hair locks treated with compositions A and B were easier to comb than the hair lock treated with composition C on humid and dry hair.

Then, the hair locks were washed again with a second shampoo and then let dried naturally (spontaneous). Better lasting curl reduction was observed while increasing the amount of cetearyl alcohol.

## Claims

1. Cosmetic composition comprising
- one or more dicarbonyl compounds corresponding to formula (I) below and/or hydrates thereof and/or salts thereof: 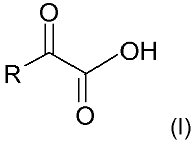 in which formula (I):
R represents an atom or group chosen from i) hydrogen, ii) carboxyl -C(O)OH, iii) linear or branched C₁-C₆ alkyl which is optionally substituted, preferably with at least one hydroxyl -OH radical, carboxyl -C(O)-OH radical or halogen radical such as Br; iv) optionally substituted phenyl, v) optionally substituted benzyl, iv) and v) preferably being optionally substituted with at least one -OH or -C(O)OH radical; vi) an indolyl radical and vii) an imidazolylmethyl radical and tautomers thereof such as 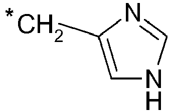 with * representing the part linked to the rest of the molecule, the dicarbonyl compounds corresponding to formula (I) and/or hydrates thereof and/or salts thereof being present in an amount ranging from 3 to 15% by weight relative to the total weight of the composition,
- one or more cationic surfactants,
- one or more non liquid and preferably solid fatty substances, the non liquid and preferably solid fatty substances being present in an amount ranging from 1% to 30% by weight relative to the total weight of the composition,
the composition having a pH of less than or equal to 4.

2. Composition according to Claim 1, in which R represents i) a hydrogen atom or ii) a linear or branched C₁-C₆ alkyl group optionally substituted with a carboxyl group.

3. Composition according to either of the preceding claims, in which the dicarbonyl compound(s) corresponding to formula (I) and/or hydrates thereof and/or salts thereof are chosen from glyoxylic acid and pyruvic acid, salts thereof and hydrates thereof, preferably from glyoxylic acid and the hydrate forms of this compound.

4. Composition according to Claim 3, in which the glyoxylic acid is in its hydrate form.

5. Composition according to any one of Claims 1 to 4, comprising from 5% to 15% by weight of one or more dicarbonyl compounds corresponding to formula (I) and/or hydrates thereof and/or salts thereof, preferably from 5% to 10% by weight relative to the total weight of the composition.

6. Composition according to any one of the preceding claims, in which the cationic surfactant(s) are chosen from:
- those corresponding to the general formula (II) below: 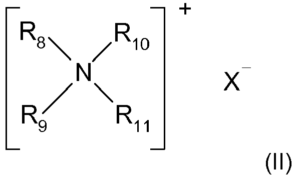
with R₈ to R₁₁, which may be identical or different, representing a linear or branched aliphatic group comprising from 1 to 30 carbon atoms, or an aromatic group such as aryl or alkylaryl, at least one of the groups R₈ to R₁₁ denoting a group comprising from 8 to 30 carbon atoms, preferably from 12 to 24 carbon atoms;
X- is an anion chosen from the group of halides, phosphates, acetates, lactates, (C₁-C₄)alkyl sulfates, (C₁-C₄) alkyl- or (C₁-C₄)alkylarylsulfonates;
- quaternary ammonium salts of imidazoline, more particularly those of formula (III) below: 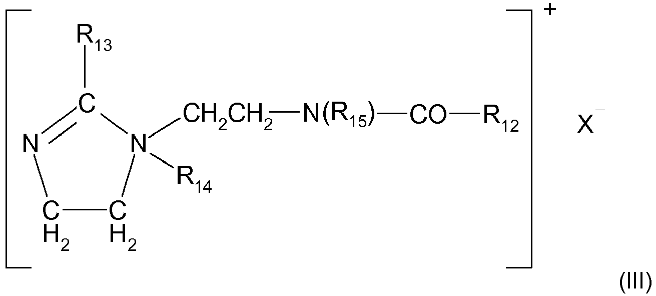 in which R₁₂ represents an alkyl or alkenyl group comprising from 8 to 30 carbon atoms, R₁₃ represents a hydrogen atom, a C₁-C₄ alkyl group or an alkyl or alkenyl group comprising from 8 to 30 carbon atoms, R₁₄ represents a C₁-C₄ alkyl group, R₁₅ represents a hydrogen atom or a C₁-C₄ alkyl group, X- is an anion chosen from the group of halides, phosphates, acetates, lactates, alkyl sulfates and alkyl- or alkylarylsulfonates, the alkyl and aryl groups of which preferably comprise from 1 to 20 carbon atoms and from 6 to 30 carbon atoms respectively;
- quaternary di- or triammonium salts, in particular of formula (IV): 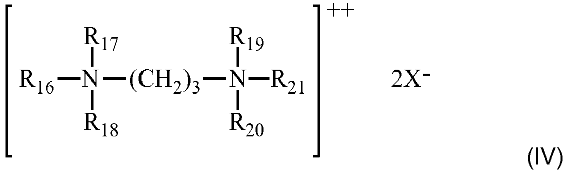
in which R₁₆ denotes an alkyl radical comprising approximately 16 to 30 carbon atoms, optionally hydroxylated and/or interrupted with one or more oxygen atoms, R₁₇ is chosen from hydrogen, an alkyl radical comprising from 1 to 4 carbon atoms or a group [(R₁₆ₐ)(R₁₇ₐ)(R₁₈ₐ)N-(CH₂)₃,
R₁₆ₐ, R₁₇ₐ, R₁₈ₐ, R₁₈, R₁₉, R₂₀ and R₂₁, which may be identical or different, are chosen from hydrogen or an alkyl radical comprising from 1 to 4 carbon atoms;
X⁻ is an anion chosen from the group of halides, acetates, phosphates, nitrates and methyl sulfates;
- quaternary ammonium salts containing at least one ester function, such as those of formula (V) below: 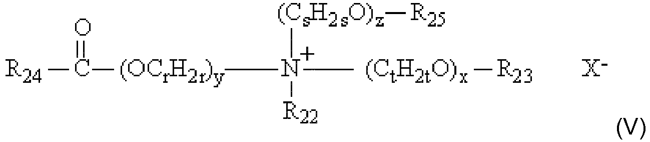 in which:
R₂₂ is chosen from C₁-C₆ alkyl and C₁-C₆ hydroxyalkyl or dihydroxyalkyl groups;
R₂₃ is chosen from: the group 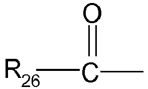
- groups R₂₇, which are linear or branched, saturated or unsaturated C₁-C₂₂ hydrocarbon-based groups,
- a hydrogen atom,
R₂₅ is chosen from: the group 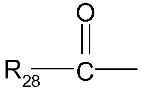
- groups R₂₉, which are linear or branched, saturated or unsaturated C₁-C₆ hydrocarbon-based groups,
- a hydrogen atom,
R₂₄, R₂₆ and R₂₈, which may be identical or different, are chosen from linear or branched, saturated or unsaturated C₇-C₂₁ hydrocarbon-based groups;
r, s and t, which may be identical or different, are integers ranging from 2 to 6;
y is an integer ranging from 1 to 10;
x and z, which may be identical or different, are integers ranging from 0 to 10;
X- is a simple or complex, organic or mineral anion;
with the proviso that the sum x + y + z is from 1 to 15, that when x is 0, then R₂₃ denotes R₂₇ and that when z is 0, then R₂₅ denotes R₂₉.

7. Composition according to any one of the preceding claims, in which the cationic surfactant(s) are chosen from cetyltrimethylammonium, behenyltrimethylammonium and dipalmitoylethylhydroxyethylmethylammonium salts, and mixtures thereof, and more particularly behenyltrimethylammonium chloride, cetyltrimethylammonium chloride, and dipalmitoylethylhydroxyethylammonium methosulfate, and mixtures thereof.

8. Composition according to one of the preceding claims, **characterized in that** the cationic surfactant(s) are present in an active material content ranging from 0.1% to 15% by weight and preferably from 0.2% to 8% by weight, preferably ranging from 0.4% to 3% by weight relative to the total weight of the composition.

9. Composition according to any one of the preceding claims, in which the weight ratio of dicarbonyl compounds corresponding to formula (I) and/or hydrate form thereof and/or salts thereof/cationic surfactants ranges from 0.1 to 20 and better still from 1 to 10.

10. Composition according to any one of the preceding claims, in which the fatty substance(s) are chosen from non-silicone fatty substances.

11. Composition according to any one of the preceding claims, in which the fatty substance(s) are chosen from fatty alcohols, esters of a fatty acid and/or of a fatty alcohol, or mixtures thereof, and more preferably from fatty alcohols.

12. Composition according to any one of the preceding claims, in which the fatty substance(s) are present in an amount ranging from 1 to 20% by weight, preferably from 5 to 20% by weight relative to the total weight of the composition.

13. Composition according to any one of the preceding claims, **characterized in that** it is aqueous and comprises water in a concentration preferably ranging from 5% to 98%, better still from 5% to 90% and even better still from 10% to 90% by weight relative to the total weight of the composition.

14. Composition according to any one of the preceding claims, **characterized in** the pH preferentially ranging from 1 to 4, preferentially from 1 to 3, better still from 1.5 to 3 and even better still from 1.7 to 3.

15. Composition according to any one of the preceding claims, **characterized in that** it results from the mixing of several compositions.

16. Process for straightening keratin fibres, especially the hair, which comprises the application to the hair of the composition according to any one of the preceding claims, and then a straightening step using a straightening iron at a temperature of at least 150°C, preferably ranging from 150 to 250°C.

17. Process according to claim 16, wherein the time of contact of the composition with the hair ranging from 10 to 60 minutes.

18. Use of the composition according to any one of Claims 1 to 15, for straightening/relaxing keratin fibres, especially the hair.

## Patentansprüche

1. Kosmetikzusammensetzung, umfassend:
- eine oder mehrere Dicarbonylverbindungen gemäß Formel (I) unten und/oder Hydrate davon und/oder Salze davon: 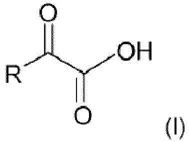 wobei in Formel (I):
R ein Atom oder eine Gruppe, ausgewählt aus den folgenden, bedeutet: i) Wasserstoff, ii) Carboxyl -C(O)OH, iii) geradkettiges oder verzweigtes C₁-C₆-Alkyl, das gegebenenfalls substituiert ist, vorzugsweise durch mindestens einen Hydroxylrest -OH, Carboxylrest -C(O)OH oder Halogenrest wie Br; iv) gegebenenfalls substituiertes Phenyl, v) gegebenenfalls substituiertes Benzyl, wobei iv) und v) vorzugsweise gegebenenfalls durch mindestens einen -OH oder -C(O)OH Rest substituiert sind; vi) einen Indolylrest und vii) einen Imidazolylmethylrest und Tautomeren davon, wie 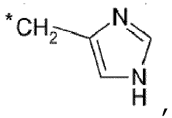 wobei * den Teil, der mit dem Rest des Moleküls verknüpft ist, bedeutet, wobei die Dicarbonylverbindungen der Formel (I) und/oder Hydrate davon und/oder Salze davon in einer Menge im Bereich von 3 bis 15 Gew.-% in Bezug auf das Gesamtgewicht der Zusammensetzung vorliegen,
- ein oder mehrere kationische Tenside,
- eine oder mehrere nichtflüssige und vorzugsweise feste Fettstoffe, wobei die nichtflüssigen und vorzugsweise festen Fettstoffe in einer Menge im Bereich von 1 Gew.-% bis 30 Gew.-% in Bezug auf das Gesamtgewicht der Zusammensetzung vorliegen,
wobei die Zusammensetzung einen pH-Wert von 4 oder weniger aufweist.

2. Zusammensetzung nach Anspruch 1, in der R i) ein Wasserstoffatom oder ii) eine geradkettige oder verzweigte C₁-C₆-Alkylgruppe, die gegebenenfalls durch eine Carboxylgruppe substituiert ist, bedeutet.

3. Zusammensetzung nach einem der vorhergehenden Ansprüche, in der die Dicarbonylverbindung(en) gemäß Formel (I) und/oder Hydrate davon und/oder Salze davon aus Glyoxylsäure und Brenztraubensäure, Salzen davon und Hydraten davon, vorzugsweise aus Glyoxylsäure und den Hydratformen dieser Verbindung, ausgewählt sin.

4. Zusammensetzung nach Anspruch 3, in der die Glyoxylsäure in ihrer Hydratform vorliegt.

5. Zusammensetzung nach einem der Ansprüche 1 bis 4, umfassend 5 Gew.-% bis 15 Gew.-% an einer oder mehreren Dicarbonylverbindungen gemäß Formel (I) und/oder Hydrate davon und/oder Salze davon, vorzugsweise 5 Gew.-% bis 10 Gew.-% in Bezug auf das Gesamtgewicht der Zusammensetzung.

6. Zusammensetzung nach einem der vorhergehenden Ansprüche, in der das kationische Tensid bzw. die kationischen Tenside aus den folgenden ausgewählt sind:
- solchen gemäß der allgemeinen Formel (II) unten: 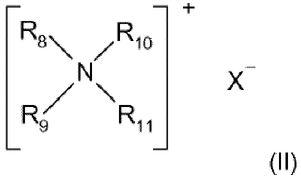 wobei R₈ bis R₁₁, die gleich oder verschieden sein können, eine geradkettige oder verzweigte aliphatische Gruppe umfassend 1 bis 30 Kohlenstoffatome oder eine aromatische Gruppe wie Aryl oder Alkylaryl bedeuten, wobei mindestens eine der Gruppen R₈ bis R₁₁ eine Gruppe umfassend 8 bis 30 Kohlenstoffatome, vorzugsweise 12 bis 24 Kohlenstoffatome, darstellt;
X⁻ ein Anion, ausgewählt aus der Gruppe der Halogenide, Phosphate, Acetate, Lactate, (C₁-C₄) -Alkylsulfate, (C₁-C₄) -Alkyl- oder (C₁-C₄)-Alkylarylsulfonate, bedeutet;
- quartären Ammoniumsalzen des Imidazolins, insbesondere denjenigen der Formel (III) unten: 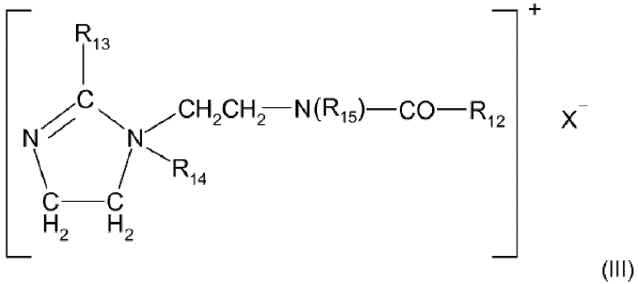 in der R₁₂ eine Alkyl- oder Alkenylgruppe umfassend 8 bis 30 Kohlenstoffatome bedeutet, R₁₃ ein Wasserstoffatom, eine C₁-C₄-Alkylgruppe oder eine Alkyl- oder Alkenylgruppe umfassend 8 bis 30 Kohlenstoffatome bedeutet, R₁₄ eine C₁-C₄-Alkylgruppe bedeutet, R₁₅ ein Wasserstoffatom oder eine C₁-C₄-Alkylgruppe bedeutet, X⁻ ein Anion, ausgewählt aus der Gruppe der Halogenide, Phosphate, Acetate, Lactate, Alkylsulfate und Alkyl- oder Alkylarylsulonate bedeutet, dessen Alkyl- und Arylgruppen vorzugsweise 1 bis 20 Kohlenstoffatome bzw. 6 bis 30 Kohlenstoffatome umfassen;
- quartären Di- oder Triammoniumsalzen, insbesondere der Formel (IV): 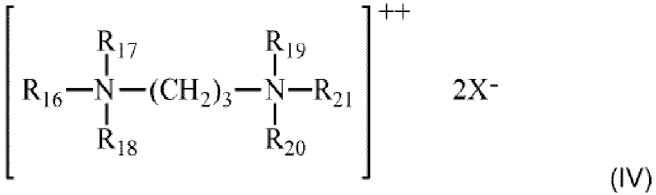 in der R₁₆ einen Alkylrest umfassend ungefähr 16 bis 30 Kohlenstoffatome bedeutet, der gegebenenfalls hydroxyliert und/oder durch ein oder mehrere Sauerstoffatome unterbrochen ist, R₁₇ aus Wasserstoff, einem Alkylrest umfassend 1 bis 4 Kohlenstoffatomen oder einer Gruppe [(R₁₆ₐ) (R₁₇ₐ) (R₁₈ₐ) N-(CH₂) ausgewählt ist,
wobei R₁₆ₐ, R₁₇ₐ, R₁₈ₐ, R₁₈, R₁₉, R_{2C} und R₂₁, die gleich oder verschieden sein können, aus Wasserstoff oder einem Alkylrest umfassend 1 bis 4 Kohlenstoffatome ausgewählt sind;
X⁻ ein Anion, ausgewählt aus der Gruppe der Halogenide, Acetate, Phosphate, Nitrate und Methylsulfate, bedeutet;
- quartären Ammoniumsalzen, die mindestens eine Esterfunktion enthalten, wie denjenigen der Formel (V) unten: 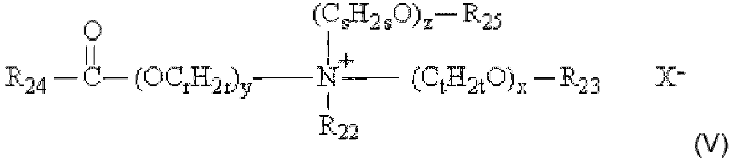 in der:
R₂₂ aus C₁-C₆-Alkyl- und C₁-C₆-Hydroxyalkyl- oder Dihydroxyalkylgruppen ausgewählt ist;
R₂₃ aus der Gruppe 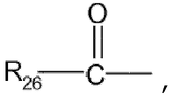
- Gruppen R₂₇, die geradkettige oder verzweigte, gesättigte oder ungesättigte C₁-C₂₂-Gruppen auf Kohlenwasserstoffbasis sind,
- einem Wasserstoffatom,
ausgewählt ist
R₂₅ aus
der Gruppe 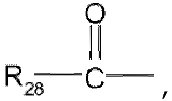
- Gruppen R₂₉, die geradkettige oder verzweigte, gesättigte oder ungesättigte C₁-C₆-Gruppen auf Kohlenwasserstoffbasis sind,
- einem Wasserstoffatom,
ausgewählt ist,
R₂₄, R₂₆ und R₂₈ die gleich oder verschieden sein können, aus geradkettigen oder verzweigten, gesättigten oder ungesättigten C₇-C₂₁-Gruppen auf Kohlenwasserstoffbasis ausgewählt sind;
r, s und t, die gleich oder verschieden sein können, ganze Zahlen im Bereich von 2 bis 6 bedeuten;
y eine ganze Zahl im Bereich von 1 bis 10 bedeutet;
x und z, die gleich oder verschieden sein können, ganze Zahlen im Bereich von 0 bis 10 bedeuten;
X⁻ ein einfaches oder komplexes organisches oder anorganisches Anion bedeutet;
mit der Maßgabe, dass die Summe x + y + z 1 bis 15 beträgt, dass, wenn x 0 bedeutet, dann R₂₃ R₂₇ darstellt und dass, wenn z 0 bedeutet, dann R₂₅ R₂₉ darstellt.

7. Zusammensetzung nach einem der vorhergehenden Ansprüche, in der das kationische Tensid/die kationischen Tenside aus Cetyltrimethylammonium-, Behenyltrimethylammonium- und Dipalmitoylethylhydroxyethylmethylammoniumsalzen und Mischungen davon, und insbesondere Behenyltrimethylammoniumchlorid, Cetyltrimethylammoniumchlorid und Dipalmitoylethylhydroxyethylammoniummethosulfate und Mischungen davon, ausgewählt ist/sind.

8. Zusammensetzung nach einem der vorhergehenden Ansprüche, **dadurch gekennzeichnet, dass** das kationische Tensid/die kationischen Tenside in einem Wirkstoffgehalt im Bereich von 0,1 Gew.-% bis 15 Gew.-% und vorzugsweise von 0,2 Gew.-% bis 8 Gew.-%, vorzugsweise im Bereich von 0,4 Gew.-% bis 3 Gew.-%, in Bezug auf das Gesamtgewicht der Zusammensetzung vorliegt/vorliegen.

9. Zusammensetzung nach einem der vorhergehenden Ansprüche, in der das Gewichtsverhältnis der Dicarbonylverbindungen gemäß Formel (I) und/oder der Hydratform davon und/oder Salzen davon zu den kationischen Tensiden im Bereich von 0,1 bis 20, noch besser 1 bis 10, liegt.

10. Zusammensetzung nach einem der vorhergehenden Ansprüche, in der der Fettstoff/die Fettstoffe aus Nichtsilikonfettstoffen ausgewählt ist/sind.

11. Zusammensetzung nach einem der vorhergehenden Ansprüche, in der der Fettstoff/die Fettstoffe aus Fettalkoholen, Estern einer Fettsäure und/oder eines Fettalkohols, oder Mischungen davon, stärker bevorzugt aus Fettalkoholen, ausgewählt ist/sind.

12. Zusammensetzung nach einem der vorhergehenden Ansprüche, in der der Fettstoff/die Fettstoffe in einer Menge im Bereich von 1 bis 20 Gew.-%, vorzugsweise 5 bis 20 Gew.-%, in Bezug auf das Gesamtgewicht der Zusammensetzung vorliegt/vorliegen.

13. Zusammensetzung nach einem der vorhergehenden Ansprüche, **dadurch gekennzeichnet, dass** sie wässrig ist und Wasser in einer Konzentration, die vorzugsweise im Bereich von 5 Gew.-% bis 98 Gew.-% noch besser 5 Gew.-% bis 90 Gew.-%, ja noch besser 10 Gew.-% bis 90 Gew.-%, in Bezug auf das Gesamtgewicht der Zusammensetzung umfasst.

14. Zusammensetzung nach einem der vorhergehenden Ansprüche, **dadurch gekennzeichnet, dass** der pH-Wert bevorzugt im Bereich von 1 bis 4 liegt, bevorzugt im Bereich von 1 bis 3 liegt, noch besser von 1,5 bis 3 liegt, ja noch besser von 1,7 bis 3 liegt.

15. Zusammensetzung nach einem der vorhergehenden Ansprüche, **dadurch gekennzeichnet, dass** sie durch Mischen von mehreren Zusammensetzungen entsteht.

16. Verfahren zum Glätten von Keratinfasern, insbesondere des Haars, umfassend das Auftragen der Zusammensetzung nach einem der vorhergehenden Ansprüche auf das Haar und anschließend einen Glättschritt unter Verwendung eines Glätteisens bei einer Temperatur von mindestens 150°C, vorzugsweise im Bereich von 150 bis 250°C.

17. Verfahren nach Anspruch 16, wobei die Kontaktzeit der Zusammensetzung mit dem Haar im Bereich von 10 bis 16 Minuten liegt.

18. Verwendung der Zusammensetzung nach einem der Ansprüche 1 bis 15 zum Glätten/Entkräusen von Keratinfasern, insbesondere des Haars.

## Revendications

1. Composition cosmétique, comprenant
- un ou plusieurs composés de dicarbonyle correspondant à la formule (I) ci-après et/ou des hydrates de ceux-ci et/ou des sels de ceux-ci 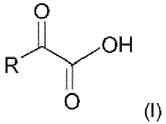
formule (I) dans laquelle
R représente un atome ou groupement choisi parmi i) hydrogène, ii) carboxyle -C(O)OH, iii) C₁-C₆-alkyle linéaire ou ramifié qui est éventuellement substitué, préférablement par au moins un radical hydroxyle -OH, un radical carboxyle -C(O)-OH ou un radical halogène tel que Br, iv) phényle éventuellement substitué, v) benzyle éventuellement substitué, iv) et v) étant préférablement éventuellement substitués par au moins un radical -OH ou -C(O)-OH, vi) un radical indolyle et vii) un radical imidazolylméthyle et des tautomères de celui-ci, tels que 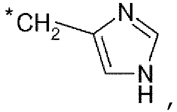 * représentant la partie liée au reste de la molécule, les composés de dicarbonyle correspondant à la formule (I) et/ou des hydrates de ceux-ci et/ou des sels de ceux-ci, étant présents selon une quantité allant de 3 à 15% en poids par rapport au poids total de la composition,
- un ou plusieurs agents tensioactifs cationiques,
- une ou plusieurs substances grasses non liquides et préférablement solides, les substances grasses non liquides et préférablement solides étant présentes selon une quantité allant de 1% à 30% en poids par rapport au poids total de la composition,
la composition ayant un pH inférieur ou égal à 4.

2. Composition selon la revendication 1, dans laquelle R représente i) un atome d'hydrogène ou ii) un groupement C₁-C₆-alkyle linéaire ou ramifié éventuellement substitué par un groupement carboxyle.

3. Composition selon l'une ou l'autre parmi les revendications précédentes, dans laquelle le ou les composés de dicarbonyle correspondant à la formule (I) et/ou des hydrates de ceux-ci et/ou des sels de ceux-ci, sont choisis parmi l'acide glyoxylique et l'acide pyruvique, les sels de ceux-ci et les hydrates de ceux-ci, préférablement parmi l'acide glyoxylique et les formes d'hydrate de ce composé.

4. Composition selon la revendication 3, dans laquelle l'acide glyoxylique se trouve sous sa forme d'hydrate.

5. Composition selon l'une quelconque des revendications 1 à 4, comprenant de 5% à 15% en poids d'un ou plusieurs composés de dicarbonyle correspondant à la formule (I) et/ou des hydrates de ceux-ci et/ou des sels de ceux-ci, préférablement de 5% à 10% en poids par rapport au poids total de la composition.

6. Composition selon l'une quelconque des revendications précédentes, dans laquelle le ou les agents tensioactifs cationiques sont choisis parmi :
- ceux correspondant à la formule générale (II) ci-auprès : 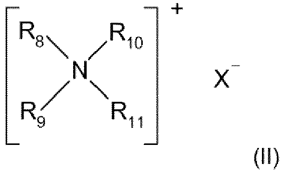
R₈ à R₁₁, qui peuvent être identiques ou différents, représentant un groupement aliphatique linéaire ou ramifié comprenant de 1 à 30 atomes de carbone, ou un groupement aromatique tel qu'aryle ou alkylaryle, au moins l'un parmi les groupements R₈ à R₁₁ désignant un groupement comprenant de 8 à 30 atomes de carbone, préférablement de 12 à 24 atomes de carbone ;
X⁻ est un anion choisi dans le groupe constitué par les halogénures, les phosphates, les acétates, les lactates, les sulfates de (C₁-C₄)alkyle, les sulfonates de (C₁-C₄) alkyle ou (C₁-C₄) alkylaryle ;
- les sels d'ammonium quaternaire d'imidazoline, plus particulièrement ceux de formule (III) ci-après : 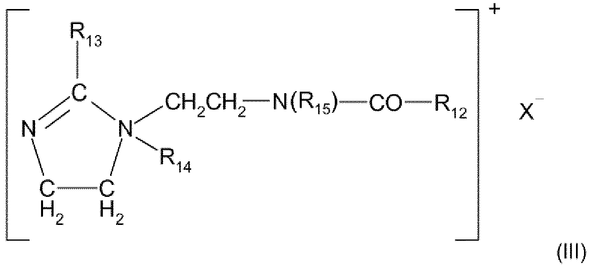 où R₁₂ représente un groupement alkyle ou alcényle comprenant de 8 à 30 atomes de carbone, R₁₃ représente un atome d'hydrogène, un groupement C₁-C₄-alkyle ou un groupement alkyle ou alcényle comprenant de 8 à 30 atomes de carbone, R₁₄ représente un groupement C₁-C₄-alkyle, R₁₅ représente un atome d'hydrogène ou un groupement C₁-C₄-alkyle, X⁻ est un anion choisi dans le groupe constitué par les halogénures, les phosphates, les acétates, les lactates, les sulfates d'alkyle et les sulfonates d'alkyle ou d'alkylaryle, les groupements alkyle et aryle de ceux-ci comprenant de préférence de 1 à 20 atomes de carbone et de 6 à 30 atomes de carbone, respectivement ;
- les sels de di- ou triammonium quaternaire, en particulier de formule (IV) : 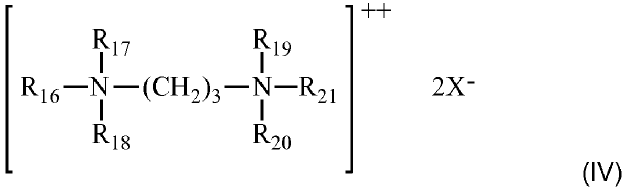
où R₁₆ représente un radical alkyle comprenant environ de 16 à 30 atomes de carbone, éventuellement hydroxylé et/ou interrompu par un ou plusieurs atomes d'oxygène, R₁₇ est choisi parmi hydrogène, un radical alkyle comprenant de 1 à 4 atomes de carbone ou un groupement [(R₁₆ₐ)(R₁₇ₐ)-(R₁₈ₐ)N-(CH₂)₃,
R₁₆ₐ, R₁₇ₐ, R₁₈ₐ, R₁₈, R₁₉, R_{2C} et R₂₁, qui peuvent être identiques ou différents, sont choisis parmi hydrogène ou un radical alkyle comprenant de 1 à 4 atomes de carbone ;
X⁻ est un anion choisi dans le groupe constitué par les halogénures, les acétates, les phosphates, les nitrates et les sulfates de méthyle ;
- les sels d'ammonium quaternaire contenant au moins une fonction ester, tels que ceux de formule (V) ci-après : 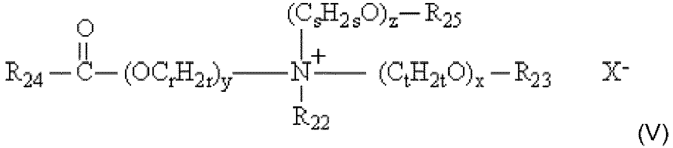 où
R₂₂ est choisi parmi des groupements C₁-C₆-alkyle et C₁-C₆-hydroxyalkyle ou dihydroxyalkyle ;
R₂₃ est choisi parmi :
le groupement 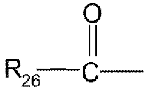
- les groupements R₂₇, qui sont des groupements hydrocarbonés en C₁-C₂₂ linéaires ou ramifiés, saturés ou insaturés,
- un atome d'hydrogène,
R₂₅ est choisi parmi :
le groupement 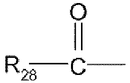
- les groupements R₂₉, qui sont des groupements hydrocarbonés en C₁-C₆ linéaires ou ramifiés, saturés ou insaturés,
- un atome d'hydrogène,
R₂₄, R₂₆ et R₂₈, qui peuvent être identiques ou différents, sont choisis parmi des groupements hydrocarbonés en C₇-C₂₁ linéaires ou ramifiés, saturés ou insaturés ;
r, s et t, qui peuvent être identiques ou différents, sont des nombres entiers allant de 2 à 6 ;
y est un nombre entier allant de 1 à 10 ;
x et z, qui peuvent être identiques ou différents, sont des nombres entiers allant de 0 à 10 ;
X⁻ est un anion simple ou complexe, organique ou minéral ;
à condition que la somme x + y + z aille de 1 à 15, que lorsque x vaut 0, alors R₂₃ désigne R₂₇ et que lorsque z vaut 0, alors R₂₅ désigne R₂₉.

7. Composition selon l'une quelconque des revendications précédentes, dans laquelle le ou les agents tensioactifs cationiques sont choisis parmi les sels de cétyltriméthylammonium, de béhényltriméthylammonium, et de dipalmitoyléthylhydroxyéthylméthylammonium, et des mélanges de ceux-ci, et plus particulièrement le chlorure de béhényltriméthylammonium, le chlorure de cétyltriméthylammonium, et le méthosulfate de dipalmitoyléthylhydroxyéthylammonium, et des mélanges de ceux-ci.

8. Composition selon l'une quelconque des revendications précédentes, **caractérisée en ce que** le ou les agents tensioactifs cationiques sont présents selon une teneur en matière active allant de z à 15% en poids et préférablement de 0,2% à 8% en poids, allant préférablement de 0,4% à 3% en poids par rapport au poids total de la composition.

9. Composition selon l'une quelconque des revendications précédentes, dans laquelle le rapport pondéral des composés de dicarbonyle correspondant à la formule (I) et/ou une forme d'hydrate de ceux-ci et/ou des sels de ceux-ci, par rapport aux agents tensioactifs cationiques, va de 0,1 à 20, et mieux encore de 1 à 10.

10. Composition selon l'une quelconque des revendications précédentes, dans laquelle la ou les substances grasses sont choisies parmi les substances grasses non siliconées.

11. Composition selon l'une quelconque des revendications précédentes, dans laquelle la ou les substances grasses sont choisies parmi les alcools gras, les esters d'un acide gras et/ou d'un alcool gras, ou des mélanges de ceux-ci, et plus préférablement parmi les alcools gras.

12. Composition selon l'une quelconque des revendications précédentes, dans laquelle la ou les substances grasses sont présentes selon une quantité allant de 1 à 20% en poids, préférablement de 5 à 20% en poids, par rapport au poids total de la composition.

13. Composition selon l'une quelconque des revendications précédentes, **caractérisée en ce qu'**elle est aqueuse et comprend de l'eau selon une concentration allant préférablement de 5% à 98%, plus préférablement de 5% à 90% et encore plus préférablement de 10% à 90% en poids, par rapport au poids total de la composition.

14. Composition selon l'une quelconque des revendications précédentes, **caractérisée en ce que** le pH va préférablement de 1 à 4, préférablement de 1 à 3, plus préférablement de 1,5 à 3 et encore plus préférablement de 1,7 à 3.

15. Composition selon l'une quelconque des revendications précédentes, **caractérisée en ce qu'**elle résulte du mélange de plusieurs compositions.

16. Procédé de lissage de fibres kératiniques, notamment des cheveux, comprenant l'application aux cheveux d'une composition selon l'une quelconque des revendications précédentes, puis une étape de lissage en utilisant un fer à lisser à une température d'au moins 150°C, allant préférablement de 150 à 250°C.

17. Procédé selon la revendication 16, dans laquelle le temps de contact de la composition avec les cheveux va de 10 à 60 minutes.

18. Utilisation de la composition selon l'une quelconque des revendications 1 à 15, pour le lissage/défrisage de fibres kératiniques, notamment des cheveux.
